# EUROPEAN PATENT SPECIFICATION

(11) **EP 1 803 429 B1**
(45) Date of publication and mention of the grant of the patent: **14.12.2011**
(21) Application number: 05793210.5
(22) Date of filing: 12.10.2005
(51) Int. Cl.: A61F 13/49, A61F 13/511, A61F 13/15, B32B 3/30, B32B 5/26, D04H 1/54

(54) **TOPSHEET FOR ABSORBENT ARTICLES**
Oberflächenschicht eines saugfähigen Artikels
FEUILLE DE SURFACE POUR ARTICLE ABSORBANT

(30) Priority: 20.10.2004 JP 2004305198
(43) Date of publication of application: 04.07.2007
(73) Proprietor: Kao Corporation, Chuo-Ku Tokyo 103-8210 (JP)
(72) Inventor: FURUTA, Kazumitsu, c/o Kao Corporation, Haga-gun, Tochigi 321-3497 (JP); MANABE, Yoko, c/o Kao Corporation, Haga-gun, Tochigi 321-3497 (JP); NAGAHARA, Shinsuke, c/o Kao Corporation, Haga-gun, Tochigi 321-3497 (JP); ISHINO, Yuichi, c/o Kao Corporation, Haga-gun, Tochigi 321-3497 (JP)
(74) Representative: Vossius & Partner
(86) International application number: PCT/JP2005/018779
(87) International publication number: WO 2006/043453

(56) References cited:
- EP-A2- 0 685 214
- EP-A2- 1 323 400
- JP-A- 3 267 058
- JP-A- 2003 275 239
- US-A- 4 659 614
- US-A- 4 886 697

## Description

### Technical Field

The present invention relates to a topsheet suited for use on the skin facing side of an absorbent article, such as a disposable diaper, a sanitary napkin, a panty liner, and an incontinence pad. The present invention also relates to a laminated nonwoven fabric.

### Background Art

Disposable diapers, sanitary napkins and the like generally include a topsheet having projections and depressions on its side to be brought into contact with a wearer (hereinafter, skin facing side). To use such a topsheet with projections and depressions reduces a contact area with the skin of a wearer, which is effective to reduce stickiness and stuffiness. For example, a topsheet composed of a bulky sheet material and another sheet material united together by embossing so as to create depressions in compressed parts and projections in non-compressed parts is known. A topsheet of this type is disadvantageous in that the properties of the projections are governed by the characteristics of the sheet materials.
Patent Document 1 discloses a surface structure of an absorbent article, comprising a liquid permeable topsheet and a hydrophilic material layer. The topsheet has a shape of a plurality of parallel ridges, and the hydrophilic material layer is in the ridges and between the topsheet and an absorbent core.
Patent Document 2 discloses a topsheet composed of two nonwoven fabric layers partly joined together by heat fusion bonding. One of the nonwoven fabric layers have projections discretely arranged in the area other than the joints.

Patent Document 1: JP 11-318983A
Patent Document 2: JP 2004-174234A

The surface structure of Patent Document 1 used with its ridges parallel to the front-to-rear direction of a wearer has the advantage of preventing side leakage. When much liquid waste such as urine is discharged, however, the liquid flows between ridges and spreads in the front-to-rear direction, which can cause leakage from the front or rear end. For example, when used in a diaper designed for settled babies who pass loose stools (a highly viscous liquid), a loose stool can flow the valleys between ridges and leak from the front or rear waist of a wearer. Even when a loose stool does not end in leakage, it broadly spreads in a specific direction and looks unpleasantly dirty during diaper change. The same problems arise when the surface structure is used as a topsheet of sanitary napkins.
The surface structure of Patent Document 1 is also designed so that the hydrophilic material layer provides the ridges with cushioning properties. Because the ridges with cushioning properties continuously extend in a specific direction, the surface structure has poor flexibility in a planar direction.
Because the projections of the topsheet of Patent Document 2 are hollow, there still is a room for improvement in liquid wicking properties.

### Disclosure of the Invention

The present invention provides a topsheet for use on the skin facing side of an absorbent article. The topsheet includes a first nonwoven fabric layer and a second nonwoven fabric layer laminated and partly joined together by heat fusion bonding, forming bonded portions (hereinafter "fusion bonds"). The first nonwoven fabric layer has a large number of projections projecting outward (toward the skin of a wearer) in portions other than the fusion bonds. The projections are arranged discretely in both a first planar direction and a second planar direction perpendicular to the first planar direction. The individual projections are filled with staple fiber. The staple fiber is substantially absent in the fusion bonds. The portion having each projection has an increasing fiber density in the order of the first nonwoven fabric layer, the staple fiber, and the second nonwoven fabric layer.

The present invention also provides laminated nonwoven fabric. The laminated nonwoven fabric includes a first nonwoven fabric layer and a second nonwoven fabric layer partly joined together by heat fusion bonding, forming fusion bonds. The first nonwoven fabric layer has a large number of projections projecting outward in portions other than the fusion bonds. The projections are arranged discretely in both a first planar direction and a second planar direction perpendicular to the first direction. The individual projections are filled with staple fiber. The staple fiber is substantially absent in the fusion bonds. The individual projections have an increasing fiber density in the order of the first nonwoven fabric layer, the staple fiber, and the second nonwoven fabric layer.

### Brief Description of the Drawings

Fig. 1 is an enlarged partial perspective of a topsheet for absorbent articles according to the present invention.
Fig. 2 schematically illustrates a process of producing the topsheet of Fig. 1.
Fig. 3 is an enlarged partial view of the first roll in Fig. 2.

### Detailed Description of the Invention

The present invention will be described based on its preferred embodiments with reference to the accompanying drawings.

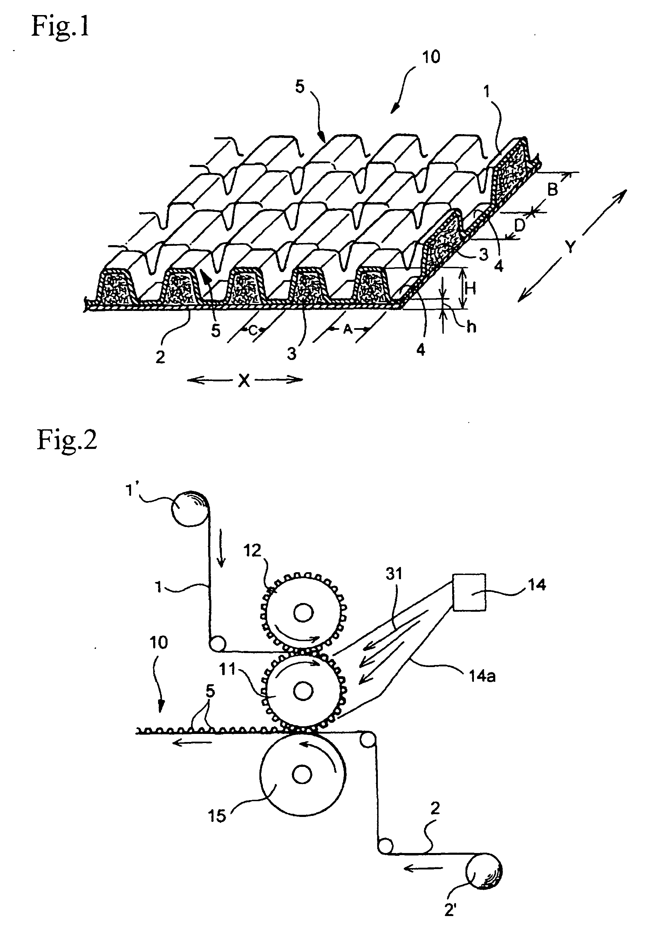

Fig. 1 is an enlarged view of a topsheet 10 for absorbent articles according to the present invention. The topsheet 10 shown in Fig. 1 is for use on the skin facing side of absorbent articles such as sanitary napkins, panty liners, incontinence pads, and the like.

The topsheet 10 is composed of a first nonwoven fabric layer 1 and a second nonwoven fabric layer 2 laminated to each other and fiber aggregates 3 composed of staple fibers discretely disposed between the nonwoven fabric layers 1 and 2. The first nonwoven fabric layer 1 defines one of surfaces of the topsheet 10 that is adapted to face the body of a wearer (skin facing side) while worn, while the second nonwoven fabric layer 2 defines the other surface that is adapted to face an absorbent member (garment facing side) while worn.

The first nonwoven fabric layer 1 and the second nonwoven fabric layer 2 are partly joined by heat fusion bonding to form numerous fusion bonds 4. The first nonwoven fabric layer 1 has its portions other than the fusion bonds 4 raised toward the wearer's skin, forming numerous projections 5.

The projections 5 are arranged discretely in both a first planar direction (direction X in Fig. 1, hereinafter referred to as direction X) and a second planar direction perpendicular to the first direction (direction Y in Fig. 1, hereinafter referred to as direction Y). Unlike the ridges of the surface structure according to Patent Document 1, the projections 5 do not have their tops connected to each other and extended in a specific direction.
Specifically, the projections 5 and the fusion bonds 4 are arranged alternately to make a line in direction X. Such lines of the alternating projections 5 and fusion bonds 4 are arrayed in direction Y. The projections 5 (and fusion bonds 4) of adjacent lines are out of alignment in direction Y. More specifically, their positions in a line and those in an adjacent line in direction X are different by a half of the pitch in direction X.

Direction X in the topsheet 10 of the present embodiment agrees with the machine direction in the production of the topsheet 10. In application to an absorbent article, the direction X of the topsheet 10 agrees with either the longitudinal direction of the absorbent article or the direction perpendicular to the longitudinal direction. The direction X in the topsheet 10 agrees with the direction of fiber orientation in each of the first and second nonwoven fabric layers. When viewed as a whole, the topsheet 10 is flat on its side of the second nonwoven fabric layer 2 and uneven on the side of the first nonwoven fabric layer 1.

The individual projections 5 of the topsheet 10 are filled with staple fibers forming fiber aggregates 3. The fiber aggregates 3 composed of staple fibers are disposed between, and in intimate contact with, the first nonwoven fabric layer 1 and the second nonwoven fabric layer 2 in portions projected to form the respective projections (hereinafter referred to as "projected portions").

The fiber aggregates 3 made out of staple fibers are arranged in the respective projected portions of the topshee 10 (i.e., the areas of the topsheet 10 where the respective projections 5 are formed). The staple fibers making up the fiber aggregates 3 are substantially absent in the fusion bonds 4. A trial made to dispose staple fibers only in the projected portions in the production of the topsheet 10 often results in unintentionally incorporating a small amount of the staple fibers into the fusion bonds. Taking this into consideration, the expression "substantially absent" is used to permit existence of such a small amount of staple fibers in the fusion bonds. Note that the percentage of the staple fiber content per unit area in the fusion bonds to that in the projected portions is not greater than 20% by weight, more preferably not greater than 5% by weight.

The fiber density in each projected portion of the topsheet 10 increases in the order of the first nonwoven fabric layer 1, the staple fiber (in the form of the fiber aggregates 3), and the second nonwoven fabric layer 2. In other words, the fiber density ρ1 of the first nonwoven fabric layer 1, the fiber density ρ2 of the fiber aggregates 3 of staple fibers, and the fiber density ρ3 of the second nonwoven fabric layer 2 have relationship: ρ1<ρ2<ρ3.
A fiber density gradient being thus established in the projected portions, liquid wastes such as urine and menstrual blood are allowed to migrate smoothly from the surface of the projections 5 to the fiber aggregates 3 and then to the second nonwoven fabric layer 2. When a large amount of a body fluid is discharged onto the topsheet 10 at a time, the topsheet 10 can retain the fluid temporarily in its projected portions because, for one thing, the first nonwoven fabric layer has an increased surface area owing to the presence of the projections 5 and, for another, the fiber aggregates 3 in the projections 5 are capable of retaining liquid.

The fiber densities ρ1, ρ2, and ρ3 respectively of the first nonwoven fabric layer 1, the staple fiber (in the form of the fiber aggregates 3), and the second nonwoven fabric layer 2 in the individual projected portions can be measured as follows.
The area ratio of the fusion bonds in the topsheet 10 is measured. The central portion of a sample is scanned into image analyzing software NewQube ver. 4.22 (from Nexus) with a CCD camera (HV-37 from Hitachi Electronics, Ltd.) and an F mount lens (Ai Af Nikol 24mm F2.8D from Nikon Corp.) using two light sources (Sunlight SL-230K2 from LPL Co., Ltd.) to capture 5 to 20 fusion bonds. The image data are binarized to specify fusion bonded portions and calculate the area ratio of the fusion bonds.

The weight per unit area of the first nonwoven fabric layer, staple fiber layer, and second nonwoven fabric layer are then measured. A 10 cm by 10 cm cut piece of the topsheet 10 is used for the measurement. The cut piece is separated into the three layers in such a manner that the fusion bonds may remain in either the first or the second nonwoven fabric layer. Application of hot air during the separating operation makes the fusion bonds remain in either one of the first and second nonwoven fabric layers more easily.
The separated layers are each weighed to the tenth of a milligram. After correction is made for the fusion bonded portions, the weight per unit area of the nonwoven fabric layer with no fusion bonds and that of the staple fiber layer are calculated. The weight per unit area of the other nonwoven fabric layer including the fusion bonds is calculated from its weight from which is subtracted the weight of the nonwoven fabric layer with no fusion bonds of the area corresponding to the total area of the fusion bonds. It should be confirmed that the staple fiber layer to be measured does not have many of the fibers thereof extending into the fusion bonds.
The first and second nonwoven fabric layers and the staple fiber layer are measured for thickness at their thickest parts in a cut area of the topsheet 10. Measurement is performed by observing a cut area under a microscope VH-8000 (from Keyence Corp.) at a magnification of 50 times. The cut area is prepared by cutting with a safety razor blade FAS-10 (from Feather Safety Razor Co. Ltd.). The cut area is scanned to record an image showing the thicknesses of the fusion bonds and projected portions before liquid absorption, followed by measuring and calculation.
The density of each layer is calculated from the thus measured weight and thickness.

It is preferred that the fiber density ρ1 of the first nonwoven fabric layer 1, the fiber density ρ2 of the staple fiber layer (fiber aggregates 3 of staple fibers), and the fiber density ρ3 of the second nonwoven fabric layer 2 have the following relationships.
The densities ρ1 and ρ3, near-fusion bond densities ρ1e and ρ3e of the nonwoven fabric layers (densities at positions near a fusion bond), and intermediate densities ρ1c and ρ3c of the nonwoven fabric layers preferably satisfy the relationships: ple<plc<pl, p3e≤p3c≤p3, and p3e<ple. When these relationships are satisfied, liquid is easily wicked away from the first nonwoven fabric layer and into the second nonwoven fabric layer near the fusion bonds, leaving the surface of the topsheet feeling dry to the touch.
It is also preferred that there be fiber missing from the staple fiber layer near the fusion bonds, or the near-fusion bond density ρ2e of the staple fiber layer be lower than the density ρ3e; that is, there is no or sparse fiber so as not to obstruct liquid migration from the first to second nonwoven fabric near the fusion bonds. It is preferred for the second nonwoven fabric layer 2 to have the relationship: ρ3e≤ρ3c=ρ3 so as to help liquid be wicked from the staple fiber layer and to prevent rewet. The term "near a fusion bond" as used herein means a position 0.5 to 1 mm away from a fusion bond in a cut area of the topsheet 10. The term "intermediate density" means a density at an intermediate position between the position of measuring the near-fusion bond density and the position of measuring the fiber density.

According to the configuration described, the topsheet 10 of the present embodiment exhibits good wicking properties due to the fiber density gradient in the projected portions and good barrier properties due to discontinuity of the projections 5. By these performance properties, urea, menstrual blood, etc. discharged on the topsheet 10 is made to be absorbed by an underlying absorbent member through a relatively small area of the topsheet 10.
Therefore, when used in an absorbent article, leakage hardly occurs from both sides and ends of the article, and the absorbent article after being soiled is less objectionable to the sight. The topsheet has excellent flexibility in planar directions and flexibly bends in both the directions X and Y. This provides a better fit to the wearer's body and allows for application to those articles and parts where flexibility is demanded.

The topsheet 10 hardly causes rewet due to the fiber density gradient in the projected portions and has a reduced contact area with the skin because of the bulkiness of the projections. As a result, the topsheet 10 effectively prevents clinging stickiness and stuffiness and gives soft and comfortable feel to the skin while in use.

Staple fibers constituting the fiber aggregates 3 being substantially absent in the fusion bonds 4, a variety of fibers of choice can be used as staple fibers to make up the fiber aggregates 3 without giving consideration to heat fusibility of fusion bonded areas. Description about the staple fibers that can be used to make the fiber aggregates 3, including preferred examples thereof, will be given later.

To enhance the above-mentioned effects, it is preferred for the topsheet 10 to have the following configuration.
The thickness H (see Fig. 1) of the projected portions is preferably 0.5 to 2.5 mm, more preferably 0.8 to 1.7 mm. The thickness h (see Fig. 1) of the fusion bonds 4 is preferably 15 to 300 µm, more preferably 20 to 150 µm. The number of the projections 5 per unit area (1 cm²) of the topsheet 10 is preferably 1 to 20, more preferably 6 to 10.

The bottom length A (see Fig. 1) of the projection 5 in direction X is preferably 0.5 to 5.0 mm, more preferably 1.0 to 4.0 mm. The bottom length B (see Fig. 1) of the projection 5 in direction Y is preferably 1.0 to 10 mm, more preferably 2.0 to 7.0 mm.
The ratio of the bottom length A in direction X to the bottom length B in direction Y (A:B) is preferably 1:1 to 1:10, more preferably 1:2 to 2:5. The bottom area of the projection 5 (bottom length A multiplied by bottom length B) is preferably 0.5 to 50 mm², more preferably 2 to 20 mm².
The lengths C and D (see Fig. 1) of the fusion bond 4 in respective directions X and Y are preferably 0.1 to 2 mm, more preferably 0.2 to 1.0 mm, and 0.2 to 5.0 mm, more preferably 0.5 to 3.0 mm, respectively.

The first and second nonwoven fabric layers can be formed of various known nonwoven fabrics, including carded nonwovens, spun-bonded nonwovens, melt-blown nonwovens, hydroentangled nonwovens, heat-rolled nonwovens, and needle-punched nonwovens. The topsheet 10 of the present embodiment uses substantially inextensible and incontractible nonwoven fabrics as the first and second nonwoven fabric layers.
From the standpoint of feel to the touch, the first nonwoven fabric layer is preferably air-through nonwoven fabric prepared by fusion-bonding fibers of a fiber web (e.g., a carded web) by air-through bonding, heat embossed nonwoven fabric, air-laid nonwoven fabric, needle-punched nonwoven fabric, etc. From the standpoint of an increased fiber density for ensuring liquid wicking from the first nonwoven fabric layer and the staple fiber layer, the second nonwoven fabric layer is preferably heat-rolled nonwoven fabric prepared by fusion-bonding fibers of a fiber web (e.g., a carded web) by use of a heat roll, heat embossed nonwoven fabric, spun-bonded nonwoven fabric, melt-blown nonwoven fabric, hydroentangled nonwoven fabric, etc.

Heat fusible fibers, particularly thermoplastic polymer fibers, are suitable as fibers constituting the first and second nonwoven fabric layers. Examples of the thermoplastic polymer include polyolefins such as polyethylene and polypropylene, polyesters such as polyethylene terephthalate, and polyamides. Conjugate fibers of sheath/core or side-by-side configuration made out of these thermoplastic polymers are also suitably useful. The first and second nonwoven fabric layers may contain fibers having no heat fusibility in addition to the heat fusible fibers.

The first nonwoven fabric layer is preferably fabricated of fibers whose fineness is 1.0 to 3.3 dtex and preferably weighs 15 to 30 g/m². The second nonwoven fabric layer is preferably fabricated of fibers whose fineness is 0.1 to 3.3 dtex and preferably weighs 10 to 30 g/m².

The staple fiber that constitutes the fiber aggregate 3 (the staple fiber filling the projections 5) can be selected from a variety of fibers with no need to take into consideration heat fusibility in fusion bonded areas. The following are examples that are preferably used.

### (1) Fiber less heat-fusion-bondable than the constituent fibers of the first and second nonwoven fabric layers (hereinafter referred to as non-heat fusible fiber)

Non-heat fusible fiber keeps freedom of movement to the pressure change during use thereby to enhance the cushioning properties of the topsheet 10.

Fiber less heat-fusion-bondable than the fibers constituting the first and second nonwoven fabric layers is, for example, a type of fiber that does not fuses at or below the melting point of the heat fusible fibers constituting the first and second nonwoven fabric layers. When in using, for example, a polyethylene resin as a main fiber component of the first and second nonwoven fabric layers, it is preferred to use, as staple fiber constituting the fiber aggregates, fibers made out of synthetic resin having a higher melting point than a polyethylene resin, such as polyethylene terephthalate (PET), polypropylene (PP), nylon, and polyamide, and semisynthetic fibers with controlled hydrophilicity, such as acetylated rayon. When in using viscous rayon, acetylated rayon or pulp as a main fiber component of the first and second nonwoven fabric layers, it is preferred to use cotton fiber, rayon fiber, etc. as staple fiber making up the fiber aggregates.

The non-heat fusible fiber preferably has a fineness of 0.5 to 6.6 dtex, more preferably 1.1 to 3.3 dtex, and a weight of 5 to 50 g/m², more preferably 10 to 30 g/m².

### (2) Self-crimping fiber

Using self-crimping fiber makes it possible to endow the projections 5 with desired properties in a step involving heat treatment during the production of the topsheet 10 or during the production of an absorbent article. For example, using self-crimping fiber showing a high degree of crimping results in the formation of projections with high elastic recovery or the formation of fiber structures having a small capillary diameter to provide a staple fiber/second nonwoven fabric layer structure with excellent liquid wicking properties.

Self-crimping fiber is a fiber having a property of shrinking on heat application while developing a three-dimensional helical crimp. In what follows, the term "self-crimping fiber" is intended to include not only self-crimping fiber before crimp development but also crimped fiber after crimp development. Examples of self-crimping fiber are conjugate fibers of sheath-core (either eccentric or concentric) or side-by-side configuration composed of two components different in shrinkages. Specific examples are described in JP 9-296325A and Japanese Patent 2759331. Examples of preferred combinations of two components (thermoplastic polymers, etc.) having different shrinkages include a combination of an ethylene-propylene random copolymer (component having high shrinkability) and polypropylene (component having low shrinkability) and a combination of polyethylene terephthalate (PET, component having low shrinkability) and polyethylene terephthalate containing an isophthalic acid unit (CoPET, component having high shrinkability).

The fiber (1) or (2) described above can be used in a proportion of 50% to 100%, preferably 80% to 100%, in the fiber aggregate 3 based on the weight of the fiber aggregate 3. The fibers that can be used in combination with the self-crimping fiber include those recited as examples of the fiber (1).

The fiber constituting the fiber aggregate 3 (the fiber filling the projection 5) is staple fibers, i.e., fibers having an average fiber length of 100 mm or less. Filling the projections 5 with staple fibers can be achieved by a method in which staple fibers are conveyed in a fluid stream such as an air stream or a method in which staple fibers are overlaid in a web form on the first nonwoven fabric layer, followed by selective removal of the fibers (e.g., removal by suction, etc. or sorting by a fluid stream). When staple fibers constituting the fiber aggregates 3 are conveyed in a fluid stream, the staple fibers preferably have an apparent length of 3 to 15 mm, more preferably 5 to 10 mm. In particular, when self-crimping fiber is used, the apparent fiber length is influential on ease of conveyance either before or after crimp development.

It is preferred that hydrophilicity of constituent fibers increase in the order of the first nonwoven fabric layer 1, the fiber aggregates 3 made of staple fibers, and the second nonwoven fabric layer 2 to improve the properties of wicking liquid away from the surface of the projections 5. The hydrophilicity of the three can be compared in terms of angle between the surface of a single fiber and a water droplet placed thereon, e.g., by spraying as measured in accordance with a contact angle measuring method.

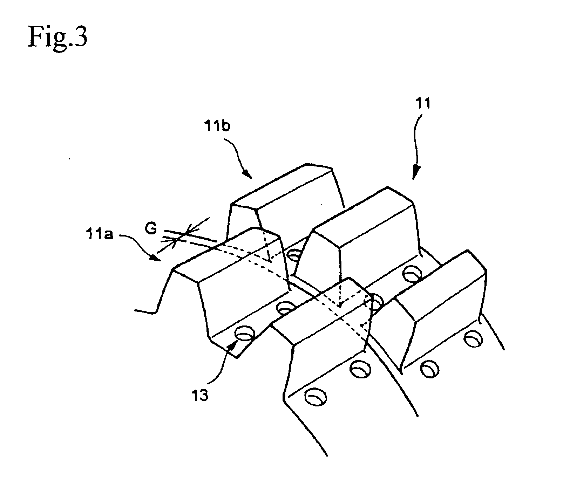

A preferred process of producing the topsheet 10 of the present embodiment will then be described by referring to Figs. 2 and 3. As illustrated in Fig. 2, a web of a first nonwoven fabric layer 1 is fed from a stock roll 1'. Separately, a web of a second nonwoven fabric layer 2 is fed from a stock roll 2'. The web of a first nonwoven fabric layer 1 is introduced into the nip between a first engraved roll 11 having projections and depressions on its surface and a second engraved roll 12 having on its surface projections and depressions mating with those of the first roll 11 to emboss the web.

Fig. 3 is an enlarged partial view of the first roll 11. The first roll 11 is an assembly of a plurality of spur gears 11 a, 11b, ... each having a given tooth width. The tooth width of each gear decides the dimension of the projections 5 in direction Y. The gears are assembled into a roll form so that their teeth are out of alignment in the axial direction by half the circular pitch. As a result, the first roll 11 has discrete projections and depressions on its peripheral surface.

A suction hole 13 is bored in every bottom land of each gear of the first roll 11. The bottom land corresponds the bottom of the depression on the surface of the first roll 11. The suction holes 13 are led to a suction source (not shown), such as a blower or a vacuum pump, and suction paths are controlled so that suction may be performed while the web of the first nonwoven fabric layer 1 is traveling from the nip line between the first and second rolls up to the meeting of the first and second nonwoven fabric layer webs. The web of the first nonwoven fabric layer 1 having been embossed between the first and the second rolls in mesh is tightly held to the surface of the first roll by the suction force exerted through the suction holes 13 and thus kept in the embossed shape. Adjacent gears are mounted with a prescribed gap G therebetween as shown in Fig. 3 so that the first nonwoven fabric layer 1 can be brought into intimate contact with the surface of the first roll 11 without imposing excessive stretching force or a cutting effect of the engagement of the two rolls to the first nonwoven fabric layer 1.

Staple fibers 31 are then fed in a fluidized form to the first nonwoven fabric layer 1 intimately held on the peripheral surface of the first roll 11 by a known fiber feeder 14. The fiber feeder 14 has a duct 14a the end of which is open to cover part of the first roll 11. The staple fibers 31 are conveyed toward the peripheral surface of the first roll 11 in an air stream generated in the duct 14a and selectively deposited in the depressions of the roll 11 by the suction through the suction holes 13. The air stream in the duct 14a can be generated by the suction through the suction holes 13 and/or any other blowing means (not shown).

The web of the second nonwoven fabric layer 2 is superposed on the web of the first nonwoven fabric layer 1 and the deposited staple fibers 31 that are still in a state suction-held to the peripheral surface of the first roll 11 as illustrated in Fig. 2. The resultant assembly web is pinched between the nip of the first roll 11 and an anvil roll 15. The anvil roll 15 or both the anvil roll 15 and the first roll 11 are previously heated to a prescribed temperature, whereby the first nonwoven fabric layer 1 and the second nonwoven fabric layer 2 are thermally bonded at the parts corresponding to the top surface of the projections on the first roll 11, i.e., the top lands of the gears.

The topsheet 10 is thus obtained. As described previously, the topsheet 10 is used in absorbent articles including disposable diapers, sanitary napkins, and incontinence pads. An absorbent article generally has a liquid permeable topsheet, a liquid impermeable backsheet, and a liquid retentive absorbent member interposed between the topsheet and the backsheet.

The above-described topsheet 10 also represents an embodiment of a laminated nonwoven fabric according to the present invention. The laminated nonwoven fabric of the invention is also useful as a member other than the topsheet in application to absorbent articles. For example, it is useful as a sheet disposed between the topsheet and the absorbent member or a sheet for forming standing gathers (standing barrier cuff), particularly a sheet forming the inner side of the barrier cuff. It also finds use in applications other than absorbent articles, for example, as a cleaning sheet for inanimate objects, particularly a wipe used mainly to absorb liquid, or a cosmetic sheet for human body. In applications as a cleaning sheet, because the projections are well conformable to a surface to be cleaned that is not smooth, the laminated nonwoven fabric is preferably used with its first nonwoven fabric layer side in contact with a surface to be cleaned. In applications as a cosmetic sheet, because the projections are well conformable to the skin and capable of absorbing excessively applied cosmetics or sweat and exert a massage effect, the laminated nonwoven fabric is preferably used with its first nonwoven fabric layer side in contact with the skin.
A preparation can be applied to the fiber sheet, which is selected according to the kind of the preparation and filled in the projections, either before filling the projections or before use. It enables the preparation to be slowly released to exert the effect of the preparation. For example, a protective agent such as wax may be applied to a cleaning sheet, or a cosmetic or a moisturizer may be applied to a cosmetic sheet, so as to be released slowly.

While the topsheet and laminated nonwoven fabric of the present invention have been described with respect to their specific embodiments, it should be understood that the invention is not limited thereto, and various changes and modifications can be made therein. For instance, while the projection 5 of the topsheet (laminated nonwoven fabric) 10 is truncated four-sided pyramidal, it may be hemispherical. The displacement of the projections 5 and the fusion bonds 4 in direction X with respect to the alignment in direction Y does not need to be a half the pitch but may be, for example 1/3 or 1/4 the pitch. The projections 5 and fusion bonds 4 of adjacent lines may be in alignment in direction Y.

### Industrial Applicability

The topsheet for absorbent articles and the laminated nonwoven fabric according to the present invention exhibit good liquid barrier properties and wicking properties, bulkiness with good cushioning properties, and high flexibility in planar directions.

## Claims

1. A laminated nonwoven fabric comprising a first nonwoven fabric layer and a second nonwoven fabric layer laminated and partly joined together by heat fusion bonding at fusion bonds,
the first nonwoven fabric layer having a large number of projections projecting outward in portions other than the fusion bonds, the projections being arranged discretely in both a first planar direction and a second planar direction perpendicular to the first planar direction,
the individual projections being filled with staple fiber, the percentage of the staple fiber content per unit area in the fusion bonds to that in the projected portions is not greater than 20% by weight, and
portions having the projections having an increasing fiber density in the order of the first nonwoven fabric layer, the staple fiber, and the second nonwoven fabric layer, the fiber density being measured with the method as described in paragraphs [0016] and [0017] of the A1-publication.

2. . The fabric according to claim 1, being a topsheet for use on the skin facing side of an absorbent article, said projections of said first nonwoven fabric layer projecting toward the skin of a wearer.

3. . The topsheet for use in an absorbent article according to claim 2, wherein the staple fiber is less heat-fusion-bondable than the constituent fibers of the first and second nonwoven fabric layers.

4. . The topsheet for use in an absorbent article according to claim 2 or 3, wherein the staple fiber is self-crimping fiber having developed a helical crimp.

## Patentansprüche

1. Mehrschichtiges Faservlies mit einer ersten Faservliesschicht und einer zweiten Faservliesschicht, die zusammenlaminiert und teilweise an Schmelzklebestellen durch Warmschweißen miteinander verbunden sind,
wobei die erste Faservliesschicht eine große Anzahl an Vorsprüngen aufweist, die in Bereichen, die außerhalb der Schmelzklebestellen liegen, vorstehen und die getrennt voneinander sowohl in einer ersten planaren Richtung als auch in einer zweiten, senkrecht zur ersten planaren Richtung liegenden planaren Richtung angeordnet sind,
wobei die einzelnen Vorsprünge mit Spinnfaser gefüllt sind, wobei der Spinnfasergehalt pro Flächeneinheit in den Schmelzklebestellen verglichen mit den vorstehenden Bereichen höchstens 20 Gew.-% beträgt, und
wobei Bereiche mit Vorsprüngen eine der Reihe nach in der ersten Faservliesschicht, der Spinnfaser und der zweiten Faservliesschicht steigende Faserdichte aufweisen, wobei die Faserdichte mit Hilfe des in den Absätzen [0016] und [0017] der A1-Veröffentlichung beschriebenen Verfahrens gemessen wird.

2. Vlies nach Anspruch 1, das die obere Lage zur Verwendung auf der zur Haut weisenden Seite eines absorbierenden Artikels bildet, wobei die Vorsprünge der ersten Faservliesschicht zur Haut des Trägers hin vorstehen.

3. Obere Lage zur Verwendung in einem absorbierenden Artikel nach Anspruch 2, wobei die Spinnfaser weniger schmelzklebbar ist als die jeweiligen Fasern der ersten und der zweiten Faservliesschicht.

4. Obere Lage zur Verwendung in einem absorbierenden Artikel nach Anspruch 2 oder 3, wobei die Spinnfaser eine selbst-kräuselnde Faser ist, die eine spiralförmige Kräuselung ausbildet.

## Revendications

1. Tissu non tissé stratifié comprenant une première couche de tissu non tissé et une deuxième couche de tissu non tissé, disposées l'une sur l'autre et partiellement assemblées par fusion à chaud sur des points de liaison, où
la première couche de tissu non tissé est pourvue d'un grand nombre d'élévations faisant saillie vers l'extérieur dans des zones autres que les points de liaison, lesdites élévations étant disposées de manière discontinue dans une première direction horizontale et dans une deuxième direction horizontale perpendiculaire à la première direction horizontale, où
chaque élévation est remplie de fibres discontinues, la masse surfacique des fibres discontinues dans les points de liaison ne dépassant pas 20% en poids de celle des parties en élévation, et où
les parties pourvues des élévations présentent une densité de fibres croissante dans l'ordre de succession suivant : première couche de tissu non tissé - fibres discontinues - deuxième couche de tissu non tissé, ladite densité de fibres étant mesurée au moyen du procédé décrit en paragraphes [0016] et [0017] de la publication Al.

2. Tissu selon la revendication 1, en tant que feuille de surface destinée à être utilisée sur la face opposée à la peau d'un article absorbant, les élévations de la première couche de tissu non tissé faisant saillie vers la peau d'un porteur.

3. Feuille de surface destinée à être utilisée dans un article absorbant selon la revendication 2, où les fibres discontinues sont moins aptes à être liées par fusion à chaud que les fibres constitutives de la première et de la deuxième couche de tissu non tissé.

4. Feuille de surface destinée à être utilisée dans un article absorbant selon la revendication 2 ou la revendication 3, où les fibres discontinues sont des fibres auto-bouclantes qui ont développé une frisure hélicoïdale.
